# EUROPÄISCHE PATENTANMELDUNG

(11) **EP 0 563 579 A1**
(43) Veröffentlichungstag der Anmeldung: **06.10.1993**
(21) Anmeldenummer: 93103058.9
(22) Anmeldetag: 26.02.1993
(51) Int. Cl.: A61F 5/04

(54) **Einteilige Bandage für das Schlüsselbein**

(30) Priorität: 02.04.1992 DE 4211023
(71) Anmelder: Bauerfeind GmbH & Co., D-47880 Kempen (DE)
(72) Erfinder: Bauerfeind, Hans B., W-4152 Kempen 1 (DE); Scheuermann, Rainer, Dr., W-2300 Kiel (DE)
(74) Vertreter: Bardehle, Heinz, Dipl.-Ing.

(57) **Zusammenfassung**

Einteilige Bandage für das Schlüsselbein mit einem den Rücken quer überspannenden Rückengurt (1), an den sich unter den Achselhöhlen verlaufende, über die Schlüsselbeine erstreckenden Schultergurte (2,3) anschließen, die sich im Bereich der Mitte des Rückengurts an einer Kreuzungsstelle (4) kreuzen und in daran anschließende Endteile (9-10) über die Hüften bis zum Bauch auslaufen, wo die Endteile mittels eines Klettendverschlusses (11) miteinander verbunden sind. Durch Ausbildung ist aus unelastischem Gurtmaterial, dessen Schultergurte (2,3) an voneinander beabstandeten Stellen auf dem Rückengurt (1) an dessen dem Rücken abgewandter Seite aufliegen, wobei die Auflagestellen (5,6) durch je einen Klettverschluß gebildet sind, dessen Kletteil (7,8) am Rückengurt (1) angebracht und dessen das jeweilige Kletteil (7,8) vollständig abdeckendes Flauschteil durch den betreffenden Schultergurt (2,3) gebildet.

## Beschreibung

Die Erfindung bezieht sich auf eine einteilige Bandage für das Schlüsselbein mit einem den Rücken quer überspannenden Rückengurt, an den sich unter den Achselhöhlen verlaufende, über die Schlüsselbeine erstreckenden Schultergurte anschließen, die sich im Bereich der Mitte des Rückengurts an einer Kreuzungsstelle kreuzen und in daran anschließende Endteile über die Hüften bis zum Bauch auslaufen, wo die Endteile mittels eines Klettendverschlusses miteinander verbunden sind.

Eine derartige Bandage als Geradehalter ist aus dem DE-GM 1, 932, 745 bekannt, d. h. als Mittel zur Haltungsverbesserung. Zu diesem Zweck besteht die Bandage aus einem elastischem Band, dessen Schultergurte sich lose über der Mitte des Rückengurts kreuzen. Aufgrund der Nachgiebigkeit des Bandes übt dieses im Falle einer Beugung des Rückens seines Trägers mit zunehmender Beugung einen größer werdenden Druck auf den Träger aus und erinnert damit gewissermaßen den Träger daran, daß er eine seiner gesunden Haltung gegenüber unerwünschter Stellung einnimmt, weshalb man eine derartige Bandage auch als "Mahnbandage" bezeichnet. Dabei ist es offenbar auch erwünscht, der Kreuzungsstelle der Schultergurte eine Verschiebemöglichkeit im Bereich des Rückengurts zu belassen, da die Schultergurte lediglich über den Rückengurt lose gelegt sind.

Die Erfindung liegt die Aufgabe zugrunde, eine einteilige Bandage zu schaffen, die durch Druck auf das Schlüsselbein eine bestimmte therapeutische Behandlung ermöglicht, z. B. bei Sprengung des Schultereckgelenkes oder bei Schlüselbeinbruch.

Hierzu ist Bandage aus unelastischem Gurtmaterial ausgebildet, dessen Schultergurte an voneinander beabstandeten Stellen auf dem Rückengurt an dessen dem Rücken abgewandter Seite aufliegen, wobei die Auflagestellen durch je einen Klettverschluß gebildet sind, dessen Kletteil am Rückengurt angebracht und dessen das jeweilige Kletteil vollständig abdeckendes Flauschteil durch den betreffenden Schultergurt gebildet ist.

Aufgrund dieser Gestaltung der Bandage läßt sich in Anpassung an das jeweils vom Patienten gegebene Körpervolumen eine gleichbleibende Spannung der Bandage erzielen, bei der sich die notwendige Länge von Rückengurt und Schultergurten dadurch automatisch ergibt, daß auf die Kletteile am Rückengurt der darüber geführte Schultergurt als Flauschteil einfach angedrückt wird, wobei sich die relative Lage des so gebildeten Klettverschlusses in bezug auf den betreffenden Schultergurt bei mehr oder minder straffem Anlegen der Bandage von selbst ergibt. Aufgrund der vollständigen Abdeckung des betreffenden Kletteils durch den zugehörigen Schultergurt kann das Kletteil gegenüber der Kleidung des Patienten keine unerwünschte Kratzwirkung hervorrufen, die die Kleidung gegebenenfalls beschädigen könnte. Mit der Schließung der Bandage durch Aneinanderdrücken des Klettendverschlusses der Endteile ergibt sich dann eine satte Anlage der gesamten Bandage an dem Körper des Patienten, wobei die dabei auf das Schlüsselbein ausgeübte Spannung ständig gleichmäßig erhalten bleibt, da die Bandage vor allem im Bereich der Schultergurte und des Rückengurtes durch die betreffenden Klettverschlüsse gesichert ist und der Klettendverschluß diese Sicherung zusätzlich gewährleistet, da der Klettendverschluß seinerseits auf die Bandage wirkende Zugspannngen mit aufnehmen kann.

Zweckmäßig bildet man die Bandage durchgehend als Flauschteil aus, das auch das Kletteil des Klettendverschlusses abdeckt. In diesem Falle besteht hinsichtlich der Schultergurte eine in der Länge unbeschränkte Anpassungsmöglichkeit der Schultergurte in bezug auf die an dem Rückengurt angebrachten Klettverschlüsse.

Eine günstige Lage der Schultergurte ergibt sich insbesondere dann, wenn die Klettverschlüsse am Rückengurt einen solchen Abstand einhalten, daß die Kreuzungsstelle der Schultergurte oberhalb des Rückengurtes, diesen nicht überlappend, liegt. In diesem Falle ergibt sich eine enge Umschlingung der Schultergurte im Bereich der Achselhöhle und des Schlüsselbeins und somit eine besonders gleichbleibende Spannung der Bandage in bezug auf das zu behandelnde Schlüsselbein.

Ein Ausführungsbeispiel ist in den Figuren dargestellt. Es zeigen:
- Figur 1: die Ansicht des Rückens eines Patienten bei angelegter Bandage,
- Figur 2: die Ansicht der Vorderseite des gleichen Patienten mit der gleichen Bandage.

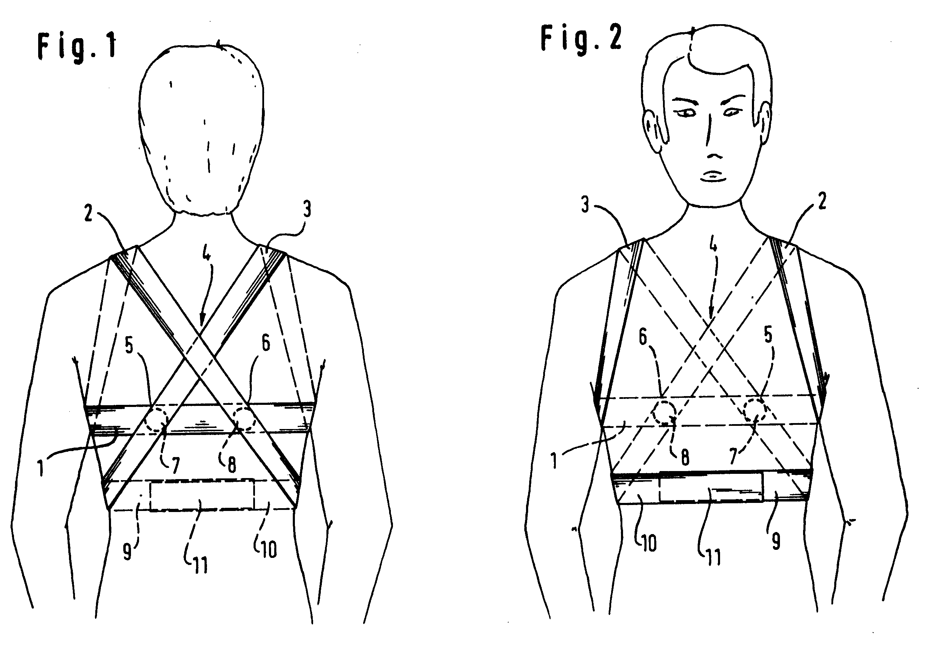

Die in den Figuren 1 und 2 dargestellte einteilige Bandage besteht aus dem den Rücken überquerenden Rückengurt 1, von dem aus unter den Achselhöhlen des dargestellten Patienten und über die Brust die beiden Schultergurte 2 und 3 zu den Schultern hochgeführt sind. Die Schultergurte 2 und 3 überqueren dann die Schultern im Bereich der Schlüsselbeine in Richtung zum Rücken und verlaufen über die auf dem Rücken liegende Kreuzungsstelle 4 zum Rückengurt 1, den sie an den Auflagestellen 5 und 6 überqueren. An den Auflagestellen 5 und 6 sind auf dem Rückengurt 1 nach außen hin die als gestrichelte Kreise angedeuteten Kletteile 7 und 8 angebracht, auf die die Innenseiten der Schultergurte 2 und 3 aufgedrückt sind. Diese Innenseiten der Schultergurte 2 und 3 bilden jeweils ein Flauschteil, in das sich das Kletteil 7 bzw. 8 einhakt und damit zwischen Rückengurt 1 und den beiden Schultergurten 2 und 3 eine feste, wenn auch abreißbare Verbindung herstellt. Im weiteren Verlauf der Schultergurte 2 und 3 von den Auflagestellen 5 und 6 ab reichen die Schultergurte 2 und 3 zu dem Hüften des Patienten und überqueren als Endteile 9 und 10 den Bauch des Patienten, bis sie sich in der Mitte des Bauchs in dem Klettendverschluß 11 zusammentreffen, der einerseits durch die nach innen weisende Flauschseite des einen Endteils und andererseits durch ein nach außen weisendes Kletteil am anderen Endteil gebildet ist (siehe insbesondere Figur 2). Die Kreuzungsstelle 4 liegt oberhalb des Rückengurts 1 an einer Stelle, wo sie den Rückengurt 1 nicht überlappt. Dies ergibt sich aus dem dargestellten Abstand zwischen den Auflagestellen 5 und 6 bzw. den Kletteilen 7 und 8, womit sich eine derart schräge Überquerung des Rückens des Patienten ergibt, daß die Schultergurte 2 und 3 relativ hoch im Bereich der Hüften diese umschlingen und dann über den Bauch des Patienten in dem Klettendverschluß 10 zusammenkommen.

Beim Anlegen der Bandage wird zunächst der Rückengurt 1 über den Rücken des Patienten gelegt, woraufhin die beiden Schultergurte 2 und 3 von der Brustseite her über die Schultern geworfen werden. Danach werden die über den Rücken des Patienten herabhängenden Schultergurte 2 und 3 mit den Händen erfaßt und, sich auf dem Rücken überkreuzend, um die Hüften geführt, um danach wie bei einem Gürtel in dem Klettendverschluß über dem Bauch zusammengefaßt zu werden. Die erforderliche Spannung der Bandage wird dabei dadurch herbeigeführt, daß einerseits die Rückengurte 2 und 3 durch Ziehen an den Endteilen 9 und 10 straff über den Rückengurt 1 gelegt werden, wobei durch Andrücken der Schultergurte 2 und 3 gegen die Kletteile 7 und 8 die gegenseitige Fixierung von Rückengurt 1 und den beiden Schultergurten 2 und 3 erfolgt. Danach wird dann unter Zug auf die beiden Endteile 9 und 10 die Spannung in den Schultergurten 2 und 3 aufrechterhalten, bis die Endteile 9 und 10 durch Schließen des Klettendverschlusses 11 miteinander verbunden werden.

Da die dargestellte Bandage aus einem unelastischem Material besteht, bleibt die beim Anlegen der Bandage eingestellte Spannung ständig gleichmäßig erhalten, womit der therapeutisch erwünschte Druck auf das jeweilige Schlüsselbein durch den betreffenden Schultergurt 2 bzw. 3 geschaffen und aufrechterhalten wird.

Die dem Körper zugewandte Seite mindestens der Schultergurte 2 und 3 und der Endteile 9 und 10 ist als Flauschteil eines Klettverschlusses ausgebildet, so daß sich in bezug auf die Kletteile 7 und 8 am Rückengurt 9 jede beliebige Länge der Schultergurte 2 und 3 bei deren Andrücken an die Kletteile 7 und 8 einstellen läßt. Die betreffenden Flauschteile decken dabei die gegenüberliegenden Kletteile einschließlich des Klettverschlusses 11 vollständig ab, so daß die Kletteile ein über der Bandage tragendes Kleidungsstück nicht beschädigen können.

## Patentansprüche

1. Einteilige Bandage für das Schlüsselbein mit einem den Rücken quer überspannenden Rückengurt (1), an den sich unter den Achselhöhlen verlaufende, über die Schlüsselbeine erstreckenden Schultergurte (2,3) anschließen, die sich im Bereich der Mitte des Rückengurts (1) an einer Kreuzungsstelle (4) kreuzen und in daran anschließende Endteile (9,10) über die Hüften bis zum Bauch auslaufen, wo die Endteile mittels eines Klettendverschlusses (11) miteinander verbunden sind, **gekennzeichnet** durch Ausbildung aus unelastischem Gurtmaterial, dessen Schultergurte (2,3) an voneinander beabstandeten Stellen auf dem Rückengurt (1) an dessen dem Rücken abgewandter Seite aufliegen, wobei die Auflagestellen (5,6) durch je einen Klettverschluß gebildet sind, dessen Kletteil (7,8) am Rückengurt (1) angebracht und dessen das jeweilige Kletteil (7,8) vollständig abdeckendes Flauschteil durch den betreffenden Schultergurt (2,3) gebildet ist.

2. Bandage nach Anspruch 1, **dadurch gekennzeichnet,** daß sie durchgehend als Flauschteil ausgebildet ist, das auch das Kletteil des Klettendverschlusses (11) abdeckt.

3. Bandage nach Anspruch 1 oder 2, **dadurch gekennzeichnet,** daß die Klettverschlüsse (7,8) am Rückengurt (1) einen derartigen Abstand einhalten, daß die Kreuzungsstelle (4) der Schultergurte (2,3) oberhalb des Rückengurts (1), diesen nicht überlappend, liegt.
